# EUROPEAN PATENT APPLICATION

(11) **EP 3 275 487 A1**
(43) Date of publication of application: **31.01.2018**
(21) Application number: 16772024.2
(22) Date of filing: 29.02.2016
(51) Int. Cl.: A61M 5/32, A61M 5/50

(54) **INJECTION NEEDLE ASSEMBLY AND DRUG INJECTION DEVICE**

(30) Priority: 27.03.2015 JP 2015065472
(71) Applicant: Terumo Kabushiki Kaisha, Tokyo 151-0072 (JP)
(72) Inventor: IWASE Yoichiro, Nakakoma-gun Yamanashi 409-3853 (JP)
(74) Representative: Casalonga
(86) International application number: PCT/JP2016/055996
(87) International publication number: WO 2016/158140

(57) **Abstract**

An injection needle assembly includes a needle tube, a needle hub holding the needle tube, a protector, an urging member, a sliding groove, and a sliding protrusion. The protector is movable between a first position of covering a needle tip of the needle tube and a second position of exposing the needle tip of the needle tube. The sliding groove is provided at one of the needle hub and the protector. The sliding protrusion is provided at the other of the needle hub and the protector and is inserted into the sliding groove to be slidable.

## Description

### Technical Field

The present invention relates to an injection needle assembly and a drug injection device used for puncturing a skin surface by a needle tip and injecting a drug into an upper layer of a skin.

### Background Art

In recent years, there has been reported that humans are infected by avian influenza. For this reason, much damage caused by a pandemic outbreak of human-to-human transmission is concerned. Here, pre-pandemic vaccines which can be effective for avian influenza have been stockpiled in the world. In addition, in order to administer pre-pandemic vaccines to many humans, a study for increasing the production volume of the vaccines has been examined.

A skin includes three parts, that is, an epidermis, a dermis, and a subcutaneous tissue. The epidermis is a layer of about 50 to 200 µm from the skin surface and the dermis is a layer of about 1.5 to 3.5 mm continuing from the epidermis. Since influenza vaccine is generally administered subcutaneously or intramuscularly, the vaccine is administered to a lower layer of the skin or a part deeper than the lower layer.

On the other hand, it has been reported that an immunity acquiring ability equivalent to subcutaneous administration or muscle administration can be obtained regardless of a decrease in dose when an influenza vaccine is administered by focusing on the upper layer of the skin having many immunocompetent cells. Therefore, since the dose can be decreased by administering the pre-pandemic vaccine to the upper layer of the skin, the pre-pandemic vaccine can be administered to more humans. In addition, the upper layer of the skin indicates the epidermis and dermis of the skin.

Various methods using a single needle, multiple needles, a patch, a gas, and the like have been reported as a method of administering a drug to the upper layer of the skin, but when considering the stability and reliability of administration and the manufacturing cost, a method using a single needle is considered to be most suitable as a method of administration to the upper layer. A Mantoux method has been known for a long time as the method of administering the vaccine to the upper layer of the skin using a single needle. In the Mantoux method, generally, a needle having a short beveled needle tip having a size of 26 to 27 gauges is inserted into the skin from an oblique direction of about 10 to 15° by 2 to 5 mm and a drug of about 100 µl is administered.

However, since it is difficult to perform the administration of drugs according to the Mantoux method, its success rate is dependent on the skill of the doctor performing the injection. In particular, since there is a possibility that children move at the time of administration, it is difficult to administer the influenza vaccine by the Mantoux method. Therefore, there has been a demand for developing a device capable of easily administering a vaccine to the upper layer of the skin.

Patent Literature 1 discloses an injection device which injects a drug to the upper layer of the skin by connecting a limiter having a skin contact surface in a needle hub. The limiter disclosed in Patent Literature 1 is provided in the periphery of a needle tube and has a gap with respect to the needle tube. A drug is administered into the skin in such a manner that a length (a protrusion length) of the needle tube protruding from a surface contacting the skin in the limiter is defined as 0.5 to 3.0 mm.

### Citation List

### Patent Literature

Patent Literature 1: JP 2001-137343 A

### Summary of Invention

### Technical Problem

However, in the injection device disclosed in Patent Literature 1, since always the needle tip of the needle tube protrudes from the surface contacting the skin in the limiter, a problem arises in that a user may be punctured by the needle tip of the needle tube by mistakes after administering the drug or upon discarding the drug injection device.

In consideration of the above-described problems, an obj ect of the invention is to provide an injection needle assembly and a drug injection device capable of preventing a user from being erroneously punctured by a needle tip of a needle tube after administering a drug or upon discarding a drug.

### Solution to Problem

In order to attain the object of the invention by solving the above-described problems, an injection needle assembly of the invention includes a needle tube that includes a needle tip capable of puncturing a living body, a needle hub that holds the needle tube, a protector, an urging member, a sliding groove, and a sliding protrusion. The protector is movable between a first position of covering the needle tip of the needle tube and a second position of exposing the needle tip of the needle tube. The urging member urges the protector toward the needle tip of the needle tube along the axial direction of the needle tube. The sliding groove is provided at one of the needle hub and the protector. The sliding protrusion is provided at the other of the needle hub and the protector and is inserted into the sliding groove to be slidable. Further, the sliding groove includes a first sliding portion, an inclined portion, and a second sliding portion. The first sliding portion extends along the axial direction of the needle tube. The inclined portion communicates with the first sliding portion and is inclined with respect to the axial direction of the needle tube and the circumferential direction of the needle tube. The second sliding portion communicates with the inclined portion and extends along the axial direction of the needle tube.

Further, the drug injection device of the invention includes an injection needle assembly and a syringe separably attached to the injection needle assembly. The above-described injection needle assembly is used as the injection needle assembly.

### Advantageous Effects of Invention

According to the injection needle assembly and the drug injection device of the invention, it is possible to prevent the puncturing of the needle tip of the needle tube after use contrary to the intention of the user.

### Brief Description of Drawings

Fig. 1 is a perspective view illustrating a drug injection device according to a first embodiment of the invention.
Fig. 2 is a cross-sectional view illustrating the drug injection device according to the first embodiment of the invention.
Fig. 3 is a cross-sectional view illustrating the drug injection device according to the first embodiment of the invention during puncturing.
Fig. 4 is a cross-sectional view illustrating the drug injection device according to the first embodiment of the invention after puncturing.
Fig. 5 is a cross-sectional view illustrating a drug injection device according to a second embodiment of the invention.
Fig. 6 is a cross-sectional view illustrating the state of the drug injection device according to the second embodiment of the invention during puncturing.
Fig. 7 is a cross-sectional view illustrating the state of the drug injection device according to the second embodiment of the invention after puncturing.

### Description of Embodiments

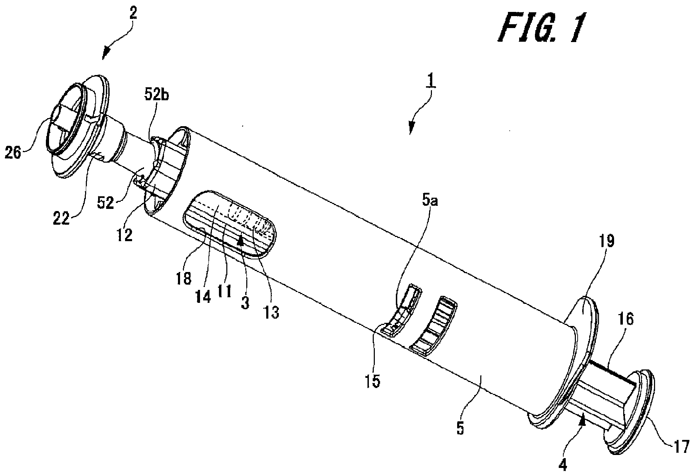

Hereinafter, embodiments of an injection needle assembly and a drug injection device of the invention will be described with reference to Figs. 1 to 7. In addition, the same reference numerals will be given to the common members in the drawings. Further, the invention is not limited to the following embodiments.

Further, a description will be made according to the following order.
1. First Embodiment
   1-1. Configuration Examples of Injection Needle Assembly and Drug Injection Device
   1-2. Drug Injection Device Usage Method
2. Second Embodiment

### <1. First Embodiment>

### 1-1. Configuration Examples of Injection Needle Assembly and Drug Injection Device

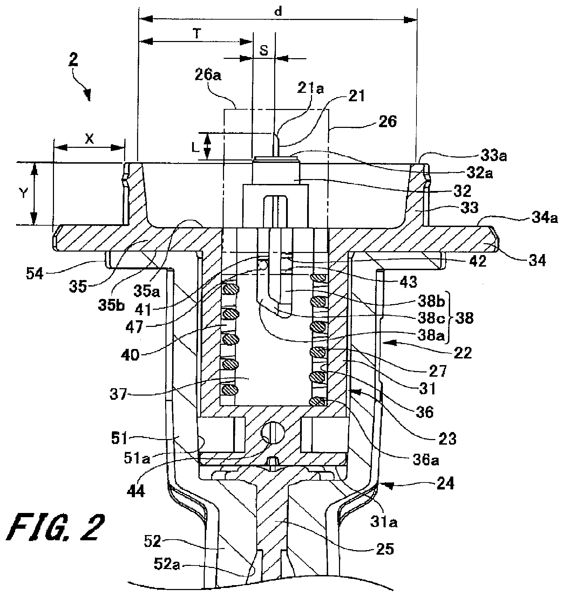

First, an injection needle assembly and a drug injection device according to a first embodiment (hereinafter, referred to as "this example") of the invention will be described with reference to Figs. 1 and 2.

Fig. 1 is a perspective view illustrating the drug injection device of this example and Fig. 2 is a cross-sectional view illustrating the injection needle assembly of this example.

A drug injection device 1 is used when puncturing a surface of a skin by a needle tip and injecting a drug into the upper layer of the skin. As illustrated in Fig. 1, the drug injection device 1 includes an injection needle assembly 2, a syringe 3 separably attached to the injection needle assembly 2, a pusher member 4, and a syringe holder 5 holding the syringe 3.

### [Syringe]

The syringe 3 is a prefilled syringe filled with a drug in advance. The syringe 3 includes a syringe body 11, a discharge portion which is provided at one end of the syringe body 11 in the axial direction, a locking mechanism 12 which is provided at the discharge portion, and a gasket 13.

The syringe body 11 is formed in a substantially hollow cylindrical shape. Further, the gasket 13 is disposed inside a cylinder hole of the syringe body 11 to be slidable. The gasket 13 is formed in a substantially columnar shape and is in close contact with the inner peripheral surface of the cylinder hole of the syringe body 11 in a liquid tight manner. Then, the gasket 13 divides a space inside the syringe body 11 into two parts. A space near the discharge portion in relation to the gasket 13 inside the syringe body 11 is formed as a liquid chamber 14 filled with a drug. Meanwhile, a plunger body 16 of the pusher member 4 to be described later is inserted into a space near the other end in relation to the gasket 13 inside the syringe body 11.

A material of the gasket 13 is not particularly limited, but an elastic material is desirable in order to satisfactorily ensure the liquid tightness with respect to the syringe body 11. Examples of the elastic material include various rubber materials such as natural rubber, isobutylene rubber, and silicone rubber, various thermoplastic elastomers such as olefins and styrenes, and mixtures thereof.

The outer diameter and the inner diameter of the syringe body 11 are appropriately set according to the use of the drug injection device 1 or the capacity of the drug stored in the liquid chamber 14. For example, in a case where the capacity of the drug stored using a general high-speed filling machine is 0.5 mL, it is desirable that the inner diameter of the syringe body 11 be set to 4.4 to 5.0 mm and the outer diameter of the syringe body 11 be set to 6.5 to 8.4 mm. Further, in a case where the capacity is 1 mL, it is desirable that the inner diameter of the syringe body 11 be set to 6.1 to 9.0 mm and the outer diameter of the syringe body 11 be set to 7.9 to 12.5 mm.

As the drug, for example, various vaccines for preventing various infectious diseases such as influenza can be mentioned, but the vaccine is not limited to the vaccine. Examples of drugs other than the vaccines include sugar injection solutions such as glucose, injection solutions for electrolyte correction such as sodium chloride and potassium lactate, vitamins, antibiotic injection solutions, contrast agents, steroid agents, proteolytic enzyme inhibition agents, lipid emulsions, anticancer agents, anesthetics, calcium heparin, antibody drugs, and the like.

A flange portion 15 is formed at the other end of the syringe body 11 in the axial direction. The flange portion 15 is locked to a locking portion 5a provided in the syringe holder 5 to be described later. Further, a discharge portion (not illustrated) is formed to be continuous to one end of the syringe body 11 in the axial direction.

The discharge portion is formed in a substantially cylindrical shape to be coaxial to the syringe body 11. Further, the cylinder hole of the discharge portion communicates with the cylinder hole of the syringe body 11. The discharge portion is formed in a tapered shape of which a diameter continuously decreases as it goes toward one end in the axial direction. When the injection needle assembly 2 is attached to the syringe 3, the front end of the discharge portion liquid-tightly contacts with the end surface of an elastic member 25 of the injection needle assembly 2 to be described later.

The discharge portion is provided with the locking mechanism 12. The locking mechanism 12 is a lure locking portion which is an example of a fixing mechanism. The locking mechanism 12 is formed in a cylindrical shape which coaxially surrounds the discharge portion. Further, the locking mechanism 12 is formed in a shape in which an inner periphery has a circular shape and an outer periphery has a hexagonal shape. The inner peripheral surface of the locking mechanism 12 is provided with a female screw portion. The female screw portion is formed to be threaded into a male screw portion 52b provided in the injection needle assembly 2.

As the material of the syringe body 11, for example, various resins such as polyvinyl chloride, polyethylene, polypropylene, cyclic polyolefin, polystyrene, poly-(4-methylpentene-1), polycarbonate, acrylic resin, acrylonitrile-butadiene-styrene copolymer, polyester such as polyethylene terephthalate, butadiene-styrene copolymer, and polyamide (for example, nylon 6, nylon 6·6, nylon 6·10, nylon 12) may be exemplified. Among these, it is desirable to use resins such as polypropylene, cyclic polyolefin, polyester, and poly-(4-methylpentene-1) from the viewpoint that molding is easy. In addition, the material of the syringe body 11 is desirably substantially transparent in order to ensure the visibility of the interior thereof.

Further, in this example, an example in which a prefilled syringe filled with a drug in advance is used as the syringe 3 has been described, but the invention is not limited thereto. For example, the syringe in which the drug is not filled into the syringe body in advance may be used.

### [Pusher Member]

The pusher member 4 includes the plunger body 16 and an operation portion 17 operating the plunger body 16. The plunger body 16 is formed in a bar shape. The plunger body 16 is inserted from an opening formed at the other end of the syringe body 11 in the axial direction into the cylinder hole of the syringe body 11. Then, one end of the plunger body 16 in the axial direction contacts with the gasket 13.

The operation portion 17 is formed at the other end of the plunger body 16 in the axial direction. The operation portion 17 is formed in a substantially disk shape. At the time of using the drug injection device 1, the operation portion 17 is pressed by a user. Accordingly, one end of the plunger body 16 in the axial direction contacts with the gasket 13 so that the gasket 13 slides toward the discharge portion.

Further, as the material of the pusher member 4, various resins exemplified as the material of the syringe body 11 can be used.

### [Syringe Holder]

Next, the syringe holder 5 will be described.

The syringe holder 5 is formed in a substantially cylindrical shape. The syringe holder 5 covers the outer peripheral surface of the syringe body 11 and the outer peripheral surface of the locking mechanism 12 of the syringe 3. Then, the syringe holder 5 can be gripped by the user when attaching the injection needle assembly 2 to the syringe 3.

A viewing window 18 is formed at one end of the syringe holder 5 in the axial direction. The viewing window 18 is provided at a position where the liquid chamber 14 of the syringe 3 can be viewed from the outside of the syringe holder 5 when the syringe 3 is attached to the syringe holder 5. Accordingly, it is possible to ensure internal visibility even when the syringe holder 5 is attached to the syringe 3.

Further, a holder flange portion 19 is formed at the other end of the syringe holder 5 in the axial direction. The holder flange portion 19 protrudes in a substantially perpendicular direction from a part of the outer peripheral surface of the syringe holder 5. Since the holder flange portion 19 is provided, it is possible to prevent a problem in which fingers gripping the syringe holder 5 slide toward the other end in the axial direction when the user administers a drug while gripping the syringe holder 5. Further, it is possible to prevent the drug injection device 1 from rolling when the drug injection device 1 is placed on a desk or a table.

Further, the locking portion 5a is provided in the middle of the syringe holder 5 in the axial direction. The locking portion 5a is an opening which penetrates the outer wall of the syringe holder 5. The flange portion 15 of the syringe 3 is locked to the locking portion 5a.

Since the syringe holder 5 is attached to the syringe 3, a diameter of the drug injection device 1 can be increased and thus the drug injection device 1 can be easily gripped. Accordingly, the operability at the time of operating the pusher member 4 is improved.

### [Injection Needle Assembly]

Next, the injection needle assembly 2 will be described.

As illustrated in Figs. 1 and 2, the injection needle assembly 2 includes a hollow needle tube 21 and a needle hub 22 holding the needle tube 21.

### [Needle Tube]

As illustrated in Fig. 2, the needle tube 21 having a size of 26 to 33 gauge (outer diameter 0.2 to 0.45 mm) according to an ISO standard of a medical needle tube (ISO 9626: 1991 / Amd. 1: 2001 (E)) is used and the needle tube having a size of 30 to 33 gauge is desirably used. The needle tube smaller than 33 gauge may be used.

One end of the needle tube 21 is provided with a needle tip 21a having a blade surface. Hereinafter, the other end of the needle tube 21 which is opposite to the needle tip 21a will be referred to as a "proximal end". A length (hereinafter, referred to as a "bevel length") in the axial direction of the needle tube 21 in the blade surface may be equal to or shorter than 1.4 mm (adult) which is the thinnest thickness of the upper layer of the skin to be described later and may be equal to or longer than about 0.5 mm which is the bevel length when a short bevel is formed in the needle tube of 33 gauge. That is, the bevel length is desirably set to a range of 0.5 to 1.4 mm.

In addition, the bevel length may be more desirably set so that the thinnest thickness of the upper layer of the skin is 0.9 mm (child) or less, that is, the bevel length is in the range of 0.5 to 0.9 mm. Further, the short bevel indicates a blade surface which is generally used in an injection needle and forms 18 to 25° with respect to the longitudinal direction of the needle.

As the material of the needle tube 21, for example, stainless steel can be exemplified, but the invention is not limited thereto. For example, aluminum, aluminum alloy, titanium, titanium alloy, and other metals can be used. Further, as the needle tube 21, not only a straight needle but also a tapered needle which is tapered in at least a part thereof may be used. As the tapered needle, a proximal end may have a larger diameter than that of a needle tip and an intermediate portion thereof may have a tapered structure. Further, the cross-sectional shape of the needle tube 21 may be not only a circular shape but also a polygonal shape such as a triangular shape.

### [Needle Hub]

The needle hub 22 includes a first member 23 that holds the needle tube 21, a second member 24 into which the discharge portion of the syringe 3 is fitted, the elastic member 25, a protector 26, and an urging member 27. The first member 23 and the second member 24 are formed as separate members. As the materials of the first member 23 and the second member 24, synthetic resins such as polycarbonate, polypropylene, and polyethylene can be exemplified.

The first member 23 is configured to include a base portion 31, an adjustment portion 32, a stable portion 33, a guide portion 34, and a support portion 37. The base portion 31 is formed in a substantially columnar shape. The base portion 31 is provided with an accommodation recess portion 36. The accommodation recess portion 36 is formed to be recessed in a substantially columnar shape from one end toward the other end of the base portion 31 in the axial direction. The accommodation recess portion 36 is provided with the support portion 37.

The support portion 37 is provided at the center portion of a bottom surface 36a of the accommodation recess portion 36 and protrudes from the bottom surface 36a of the accommodation recess portion 36 toward the axial direction of the base portion 31. The support portion 37 is formed in a substantially columnar shape. The side surface of the support portion 37 is provided with a sliding groove 38.

The sliding groove 38 includes a first sliding portion 38a, a second sliding portion 38b, and an inclined portion 38c which communicates with the first sliding portion 38a and the second sliding portion 38b. The first sliding portion 38a, the second sliding portion 38b, and the inclined portion 38c are groove portions which are recessed inward in the radial direction from the side surface of the support portion 37. Further, a length extending inward in the radial direction from the side surface of the support portion 37 in each of the first sliding portion 38a and the inclined portion 38c, that is, a depth of the groove is set to be shallower than a depth of the groove of the second sliding portion 38b. Then, a sliding protrusion 47 of the protector 26 to be described later is inserted into the first sliding portion 38a, the second sliding portion 38b, and the inclined portion 38c to be slidable.

The first sliding portion 38a and the second sliding portion 38b are formed in parallel to the axial direction of the support portion 37 from one end toward the other end of the support portion 37 in the axial direction. Further, the first sliding portion 38a and the second sliding portion 38b are provided to have a predetermined gap therebetween in the circumferential direction of the support portion 37.

Further, the inclined portion 38c is formed to be continuous to the other end of each of the first sliding portion 38a and the second sliding portion 38b in the axial direction of the support portion 37. In the sliding groove 38 of this example, a length of the second sliding portion 38b extending in the axial direction of the support portion 37 is set to be longer than a length of the first sliding portion 38a extending in the axial direction of the support portion 37. For that reason, an end near the first sliding portion 38a in the inclined portion 38c is located at one side in the axial direction of the support portion 37 in relation to an end near the second sliding portion 38b. That is, the inclined portion 38c is inclined from one side toward the other side in the axial direction of the support portion 37 as it goes from the first sliding portion 38a toward the second sliding portion 38b in the circumferential direction of the support portion 37. For that reason, the inclined portion 38c is inclined with respect to the axial direction and the circumferential direction of the support portion 37.

One end of the first sliding portion 38a in the axial direction of the support portion 37 is provided with a first stopper 41. The first stopper 41 is a protrusion which protrudes outward in the radial direction of the support portion 37 from the bottom surface of the first sliding portion 38a. The sliding protrusion 47 of the protector 26 to be described later contacts with the first stopper 41.

One end of the second sliding portion 38b in the axial direction of the support portion 37 is provided with a second stopper 42 and a return regulation portion 43. The second stopper 42 and the return regulation portion 43 are protrusions which protrude in the radial direction of the support portion 37 from the bottom surface of the second sliding portion 38b. The return regulation portion 43 is provided at the other side in the axial direction of the support portion 37 in relation to the second stopper 42. Further, the other end surface of the return regulation portion 43 in the axial direction of the support portion 37 is formed as an inclined surface and one end surface in the axial direction is uprightly formed in a substantially perpendicular direction from the bottom surface of the second sliding portion 38b. The sliding protrusion 47 of the protector 26 to be described later contacts with the second stopper 42 and the return regulation portion 43.

Further, one end surface in the axial direction of the support portion 37 is provided with the adjustment portion 32 and the adjustment portion 32 is formed as a columnar convex portion which protrudes in the axial direction of the support portion 37. The axis of the adjustment portion 32 matches the axes of the base portion 31 and the support portion 37.

A penetration hole through which the needle tube 21 passes is provided at the axes of the base portion 31, the support portion 37, and the adjustment portion 32. The base portion 31 is provided with an injection hole 44 for injecting an adhesive into a penetration hole. The injection hole 44 is opened to the outer peripheral surface of the base portion 31 and communicates with the penetration hole. That is, the needle tube 21 is fixed to the base portion 31 and the support portion 37 by the adhesive injected from the injection hole 44 into the penetration hole.

The proximal end of the needle tube 21 protrudes from the other end surface 31a of the base portion 31 in the axial direction. The base portion 31 is inserted from the end surface 31a into the second member 24 and the proximal end of the needle tube 21 is inserted through the insertion hole of the elastic member 25. Then, the end surface 31a of the base portion 31 contacts with the end surface of the elastic member 25.

Further, the outer peripheral surface of the base portion 31 is provided with a connection piece 35. The connection piece 35 is formed as an annular flange portion which protrudes outward in the radial direction of the base portion 31 at one end of the base portion 31 in the axial direction . The connection piece 35 includes flat surfaces 35a and 35b which face each other in the axial direction of the base portion 31. The second member 24 is connected to the flat surface 35b of the connection piece 35. Further, a front end of the connection piece 35 is formed as the guide portion 34. The guide portion 34 will be described in detail later.

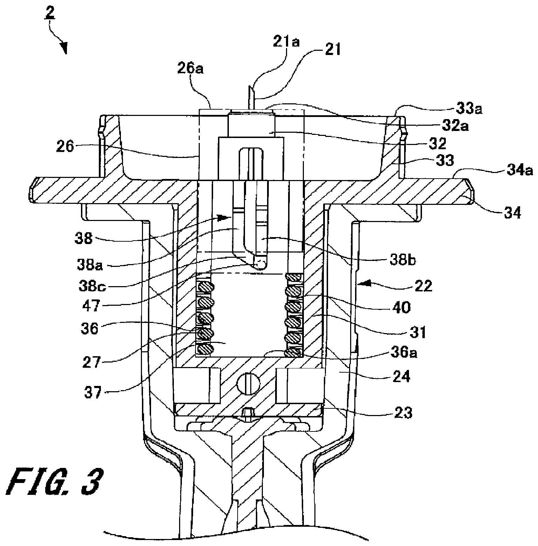

An end surface of the adjustment portion 32 is formed as a needle protruding surface 32a from which the needle tip 21a of the needle tube 21 protrudes. The needle protruding surface 32a is formed as a flat surface which is orthogonal to the axial direction of the needle tube 21. The needle protruding surface 32a defines a depth of puncturing a skin by the needle tube 21 while contacting a skin surface at the time of puncturing the upper layer of the skin by the needle tube 21. That is, a depth in which the needle tube 21 punctures the upper layer of the skin is determined by a length (hereinafter, referred to as a "protrusion length L") of the needle tube 21 protruding from the needle protruding surface 32a (see Fig. 3).

A thickness of the upper layer of the skin corresponds to a depth from the surface of the skin to the dermis layer and is substantially in the range of 0.5 to 3. 0 mm. For that reason, the protrusion length L of the needle tube 21 can be set to a range of 0.5 to 3.0 mm.

Incidentally, the administration site of the influenza vaccine is generally a deltoid muscle. Here, for 19 children and 31 adults, the thickness of the upper layer of the skin of the deltoid muscle was measured. This measurement was carried out by imaging the upper layer of the skin with high ultrasonic reflectivity using an ultrasonic measuring apparatus (NP 60R-UBM echo having high resolution for small animals, Nepa Gene Co., Ltd.). Since the measured values had a log-normal distribution, a range of MEAN ±2 SD was obtained by geometric mean.

As a result, the thickness of the upper layer of the skin of the deltoid muscle of the child was 0.9 to 1. 6 mm. Further, the thickness of the upper layer of the skin of the deltoid muscle of the adult was 1.4 to 2.6 mm at the distal portion, 1.4 to 2.5 mm at the center portion, and 1.5 to 2.5 mm at the proximal portion. From the description above, it was confirmed that the thickness of the upper layer of the skin of the deltoid muscle was 0.9 mm or more for the child and was 1.4 mm or more for the adult. Thus, it is desirable to set the protrusion length L of the needle tube 21 to a range of 0.9 to 1.4 mm regarding the injection at the upper layer of the skin of the deltoid muscle.

When the protrusion length L is set in this way, the blade surface of the needle tip 21a can be located at the upper layer of the skin. As a result, the needle hole (the drug discharge opening) opened to the blade surface is located at the upper layer of the skin even when the needle hole is located at any position inside the blade surface. In addition, if the needle tip 21a is inserted to be deeper than the upper layer of the skin even when the drug discharge opening is located at the upper layer of the skin, the drug flows to a subcutaneous tissue from a gap between the cut skin and the side surface of the end of the needle tip 21a. For this reason, it is important to reliably position the blade surface at the upper layer of the skin.

In addition, in the case of the administration to the upper layer of the skin, it is difficult to set the bevel length to 1.0 mm or less in the needle tube which is thicker than 26 gauge. Thus, it is desirable to use the needle tube which is thinner than 26 gauge in order to set the protrusion length L of the needle tube 21 to a desirable range (0.9 to 1.4 mm).

The needle protruding surface 32a of the adj ustment portion 32 is formed so that a distance S from the peripheral edge to the outer peripheral surface of the needle tube 21 becomes 1.4 mm or less and desirably in the range of 0.3 to 1.4 mm. The distance S from the peripheral edge of the needle protruding surface 32a to the peripheral surface of the needle tube 21 is set in consideration of the pressing force applied to the blisters formed by administering the drug to the upper layer of the skin. That is, the size of the needle protruding surface 32a is set so that the needle protruding surface is sufficiently smaller than the blister formed on the upper layer of the skin and does not disturb the formation of the blister. As a result, the leakage of the administered drug can be prevented even when the needle protruding surface 32a is pressed against the skin around the needle tube 21.

Next, the protector 26 will be described. As illustrated in Figs. 1 and 2, the protector 26 covers the peripheries of the adjustment portion 32 through which the needle tube 21 passes and the needle tip 21a of the needle tube 21 in a state before the skin is punctured by the needle tube 21. The protector 26 is formed in a cylindrical shape.

The protector 26 is supported by the support portion 37 to be movable in the axial direction of the support portion 37 (the axial direction of the needle tube 21) and to be rotatable in the circumferential direction (the circumferential direction of the needle tube 21). Then, a part of the protector 26 at the other side in the axial direction is inserted into a space 40 formed between the accommodation recess portion 36 and the support portion 37.

The sliding protrusion 47 is formed at the inner wall of the cylinder hole of the protector 26. The sliding protrusion 47 protrudes inward in the radial direction of the protector 26 from the inner wall of the protector 26. The sliding protrusion 47 is inserted into the sliding groove 38 to be slidable.

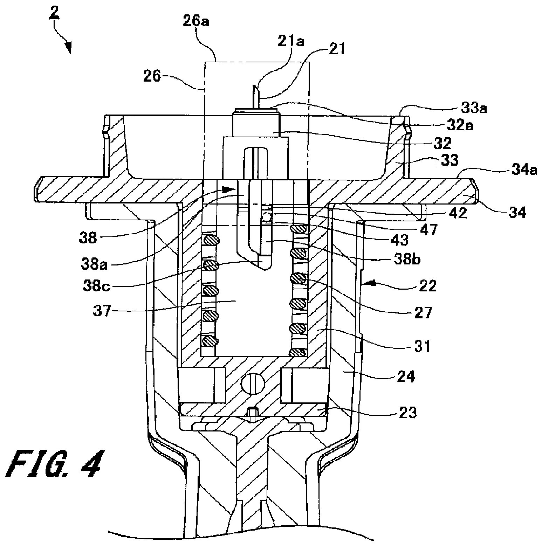

In a state before puncturing, the sliding protrusion 47 is located at the first sliding portion 38a of the sliding groove 38. In order to puncture the skin by the needle tube 21, the sliding protrusion 47 is located at the inclined portion 38c of the sliding groove 38 (see Fig. 3). Then, after puncturing, the sliding protrusion 47 is located between the second stopper 42 and the return regulation portion 43 in the second sliding portion 38b of the sliding groove 38 (see Fig. 4).

The shape of the protector 26 is not limited to a cylindrical shape and may be, for example, formed in a square tubular shape such as a quadrangular prism or a hexagonal prism having a cylindrical hole at the center.

In addition, the number of the sliding protrusions 47 is not limited to one for the protector 26 and two or more sliding protrusions 47 may be provided in the inner wall of the protector 26. When the sliding protrusion 47 is provided at a plurality of positions of the protector 26, it is desirable to provide the sliding groove 38 at a plurality of positions corresponding to the number of the sliding protrusions 47 in the support portion 37. If the sliding protrusion 47 and the sliding groove 38 are provided at a plurality of positions, it is possible to smoothly move and rotate the protector 26 without rattling when the protector 26 moves in the axial direction of the support portion 37 and rotates in the circumferential direction of the support portion 37.

It is desirable to set the thickness of the protector 26 to be sufficiently smaller than the diameter of the needle protruding surface 32a in order to prevent the disturbance of the formation of blisters.

The stable portion 33 is formed in a cylindrical shape protruding from the flat surface 35a of the connection piece 35. The needle tube 21, the adjustment portion 32, and the protector 26 are disposed inside the cylinder hole of the stable portion 33. That is, the stable portion 33 is formed in a cylindrical shape through which the needle tube 21 passes and which covers the peripheries of the adjustment portion 32 and the protector 26 and is formed to be separated from the needle tip 21a of the needle tube 21 in the radial direction.

An end surface 33a of the stable portion 33 is located at the proximal end side of the needle tube 21 in relation to the needle protruding surface 32a of the adjustment portion 32. When the living body is punctured by the needle tip 21a of the needle tube 21, the needle protruding surface 32a first contacts the skin surface and then the end surface 33a of the stable portion 33 contacts the skin surface. At this time, since the end surf ace 33a of the stable portion 33 contacts the skin, the drug injection device 1 is stabilized and thus the needle tube 21 can be held in a posture substantially perpendicular to the skin.

In addition, even when the end surface 33a of the stable portion 33 is located on the same plane as that of the needle protruding surface 32a or is located near the needle tip 21a of the needle tube 21 in relation to the needle protruding surface 32a, it is possible to maintain a posture in which the needle tube 21 is substantially perpendicular to the skin. Considering the rising of the skin when the stable portion 33 is pressed against the skin, the axial distance between the needle protruding surface 32a and the end surface 33a of the stable portion 33 is desirably set to 1.3 mm or less.

Further, an inner diameter d of the stable portion 33 is set to be equal to or larger than the diameter of the blister formed on the skin. Specifically, the inner diameter is set so that a distance T from the inner wall surface of the stable portion 33 to the peripheral edge of the needle protruding surface 32a becomes in the range of 4 mm to 15 mm. Accordingly, it is possible to prevent the disturbance of the formation of the blister without any pressure applied from the inner wall surface of the stable portion 33 to the blister.

Particularly, there is no upper limit as long as the distance T from the inner wall surface of the stable portion 33 to the needle protruding surface 32a is 4 mm or more . However, when the distance T is set to be large, the outer diameter of the stable portion 33 increases. For this reason, in a case where the needle tube 21 punctures a thin arm like a child, the entire end surface 33a of the stable portion 33 hardly contacts the skin. For that reason, it is desirable to set the distance T to 15 mm to maximum when considering the thin arm of the child.

When the distance S from the needle protruding surface 32a to the outer peripheral surface of the needle tube 21 is 0.3 mm or more, the adjustment portion 32 does not enter the skin. Thus, the inner diameter d of the stable portion 33 can be set to 9 mm or more when considering the distance T (4 mm or more) from the inner wall surface of the stable portion 33 to the peripheral edge of the needle protruding surface 32a and the diameter (about 0.3 mm) of the needle protruding surface 32a.

In addition, the shape of the stable portion 33 is not limited to a cylindrical shape and may be, for example, formed in a square tubular shape such as a quadrangular prism or a hexagonal prism having a cylindrical hole at the center.

The guide portion 34 is a front end side portion which is located at the outside of the first member 23 in the radial direction in relation to the stable portion 33 of the connection piece 35. The guide portion 34 includes a contact surface 34a which contacts a skin. The contact surface 34a is a part of the flat surface 35a of the connection piece 35 and is a flat surface which is substantially parallel to the end surface 33a of the stable portion 33. When the stable portion 33 is pressed until the contact surface 34a of the guide portion 34 contacts the skin, a force in which the stable portion 33 and the needle tube 21 press the skin can be ensured to a predetermined value or more at all times. Accordingly, a portion (corresponding to the protrusion length L) protruding from the needle protruding surface 32a of the needle tube 21 reliably punctures the skin.

A distance (hereinafter, referred to as a "guide portion height") Y from the contact surface 34a of the guide portion 34 to the end surface 33a of the stable portion 33 is set so that the needle tube 21 and the stable portion 33 can puncture the skin while pressing the skin at an appropriate pressing force. In addition, the appropriate pressing forces of the needle tube 21 and the stable portion 33 are, for example, 3 to 20 N. Accordingly, when the pressing forces applied from the needle tube 21 and the stable portion 33 to the skin are guided by the guide portion 34, the needle tip 21a of the needle tube 21 can be reliably located at the upper layer of the skin and the user can feel safe.

A guide portion height Y is appropriately determined on the basis of the inner diameter d of the stable portion 33 and a length (hereinafter, referred to as a "guide portion length") X from the front end surface of the guide portion 34 to the outer peripheral surface of the stable portion 33. For example, when the inner diameter d of the stable portion 33 is 12 mm and the guide portion length X is 3.0 mm, the guide portion height Y is set to a range of 2.3 to 6.6 mm.

Further, the urging member 27 is disposed in the space 40 and is interposed between the protector 26 and the bottom surface 36a of the accommodation recess portion 36. Then, the urging member 27 is disposed to cover the periphery of the support portion 37.

The urging member 27 is a compression coil spring and urges the protector 26 toward one side in the axial direction, that is, the needle tip 21a of the needle tube 21. The urging force of the urging member 27 is set to be smaller than an appropriate pressing force when puncturing the skin by the needle tube 21 and is set to, for example, 3 N or less. Accordingly, since the urging force of the urging member 27 does not disturb the positioning of the needle tip 21a of the needle tube 21 at the upper layer of the skin when the needle tube 21 punctures the skin, the needle tip 21a of the needle tube 21 can be reliably located at the upper layer of the skin.

Further, in this example, an example in which the compression coil spring is used as the urging member 27 has been described, but the invention is not limited thereto. As the urging member, an elastic member that is elastically deformed by a predetermined pressure applied thereto may be used. For example, other various spring members such as leaf springs, sponges, gels, and rubber members can be used.

### [Second Member]

Next, the second member 24 will be described. The second member 24 is formed in a cylindrical shape. One end of the second member 24 in the axial direction is formed as an insertion portion 51 into which the base portion 31 of the first member 23 is inserted and the other end thereof is formed as a fitting portion 52 into which the discharge portion of the syringe 3 is fitted. The cylinder hole 51a of the insertion portion 51 is set to a size corresponding to the base portion 31 of the first member 23.

Afixedpiece54 isprovidedat the outer peripheral surface of one end of the second member 24 of the insertion portion 51 in the axial direction. The fixedpiece 54 is formed as an annular flange which protrudes outward in the radial direction and is continuous to the front end of the insertion portion 51. The flat surface 35b of the connection piece 35 provided in the first member 23 is fixed to the fixed piece 54 while being in contact therewith. As a method of fixing the fixed piece 54 and the connection piece 35 to each other, for example, adhesives, ultrasonic welding, laser welding, fixing screws, and the like can be exemplified.

The outer diameter of the fitting portion 52 is set to be smaller than the outer diameter of the insertion portion 51. Further, a cylinder hole 52a of the fitting portion 52 is set to a size corresponding to the discharge portion of the syringe 3 and the diameter continuously decreases as it goes toward the insertion portion 51. Further, the outer peripheral surface of the fitting portion 52 is provided with the male screw portion 52b to be threaded into the locking mechanism 12 of the syringe 3 (see Fig. 1). Further, the elastic member 25 is disposed between the cylinder hole 51a of the insertion portion 51 and the cylinder hole 52a of the fitting portion 52.

### [Elastic Member]

Next, the elastic member 25 will be described. Theelastic member 25 is made of an elastically deformable member. As the material of the elastic member 25, for example, various rubber materials such as natural rubber, silicone rubber, and isobutylene rubber, various thermoplastic elastomers such as polyurethanes and styrenes, or a mixture thereof can be exemplified.

The elastic member 25 is disposed inside the second member 24 and is interposed between the first member 23 and the syringe 3. Then, a gap formed between the second member 24 and the outer peripheral surface of the proximal end side of the needle tube 21 protruding from the first member 23 is filled. Then, since the elastic member 25 is elastically deformed when the discharge portion of the syringe 3 is fitted into the second member 24, the elastic member 25 liquid-tightly adheres to the outer peripheral surface of the needle tube 21. Accordingly, it is possible to prevent a problem in which the drug filled in the syringe 3 enters between the needle tube 21 and the elastic member 25 and leaks toward the first member 23.

### 1-2. Drug Injection Device Usage Method

Next, a method of using the drug injection device 1 with the above-described configuration will be described with reference to Figs. 1 to 4.

Fig. 3 is a cross-sectional view illustrating a main part of the drug injection device 1 during puncturing and Fig. 4 is a cross-sectional view illustrating a main part of the drug injection device 1 after puncturing.

First, as illustrated in Figs. 1 and 2, the syringe 3 is attached to the injection needle assembly 2 in advance. Specifically, the discharge portion of the syringe 3 is inserted into the fitting portion 52 of the secondmember 24 and the locking mechanism 12 is threaded into the male screw portion 52b. Accordingly, the attachment of the injection needle assembly 2 with respect to the syringe 3 is completed.

At this time, the sliding protrusion 47 of the protector 26 is located at the first sliding portion 38a of the sliding groove 38. Further, the protector 26 is urged toward one end in the axial direction by the urging member 27, but the sliding protrusion 47 contacts with the first stopper 41. Accordingly, it is possible to prevent the separation of the protector 26 from the support portion 37 of the first member 23.

Further, the needle tip 21a of the needle tube 21 is covered by the protector 26 and is accommodated inside the cylinder hole of the protector 26. Accordingly, it is possible to prevent the puncturing of the needle tip 21a of the needle tube 21 contrary to the intension of the user in a state before puncturing. In addition, the position of the protector 26 at this time is set as a first position.

Next, the end surface 33a of the stable portion 33 faces the skin. Accordingly, the needle tip 21a of the needle tube 21 faces the skin to be punctured. Next, the drug injection device 1 is moved in a direction substantially perpendicular to the skin. Accordingly, a front end surface 26a of the protector 26 is pressed against the skin. Further, when the drug injection device 1 is pressed against the skin while resisting the urging force of the urging member 27, the sliding protrusion 47 of the protector 26 slides along the first sliding portion 38a.

Then, the protector 26 moves along the axial direction of the support portion 37, that is, the axial direction of the needle tube 21 and is inserted into the space 40. Accordingly, the needle protruding surface 32a of the adjustment portion 32 and the needle tip 21a of the needle tube 21 protrude and the needle tip 21a of the needle tube 21 is exposed from one end of the protector 26 in the axial direction, that is, the front end surface 26a. The position of the protector 26 at this time is set as a second position.

Further, the drug injection device 1 is moved in a direction substantially perpendicular to the skin so that the needle tip 21a punctures the skin and the end surface 33a of the stable portion 33 is pressed against the skin. At this time, since the needle protruding surface 32a contacts the skin, the skin can be flatly deformed and the needle tip 21a of the needle tube 21 can puncture the skin by the protrusion length L.

Next, the end surface 33a of the stable portion 33 is pressed until the contact surface 34a of the guide portion 34 contacts the skin. Here, the length of the guide portion height Y (see Fig. 2) is set so that the needle tube 21 and the stable portion 33 can puncture the skin at an appropriate pressing force. For that reason, a force of pressing the skin by the stable portion 33 becomes a predetermined value.

As a result, an appropriate pressing force of the stable portion 33 can be recognized by the user and the needle tip 21a of the needle tube 21 and the blade surface can be reliably located at the upper layer of the skin. In this way, since the guide portion 34 serves as a mark for recognizing the appropriate pressing force of the stable portion 33, the user can safely use the drug injection device 1.

Further, since the stable portion 33 contacts with the skin, the posture of the drug injection device 1 is stabilized and the needle tube 21 can straightly puncture the skin. Further, since it is possible to prevent a blur occurring in the needle tube 21 after puncturing, it is possible to stably administer the drug.

Furthermore, for example, in the needle tube of the protrusion length which is very short to be about 0.5 mm, there is a case where the needle tip cannot be inserted into the skin even when contacting with the skin. However, since the skin pressed by the stable portion 33 is pressed downward in the perpendicular direction, the skin inside the stable portion 33 is pulled and a tension is applied to the skin. For that reason, the skin hardly escapes from the needle tip 21a of the needle tube 21. Thus, since the stable portion 33 is provided, it is possible to obtain an effect in which the needle tip 21a can be more easily inserted into the skin.

Further, as illustrated in Fig. 3, the sliding protrusion 47 passes through the inclined portion 38c from the first sliding portion 38a and slides to the second sliding portion 38b. Here, the inclined portion 38c is inclined with respect to the axial direction of the support portion 37. For that reason, when the sliding protrusion 47 moves in the inclined portion 38c, the protector 26 rotates by a predetermined angle about the axis of the support portion 37 in the circumferential direction of the support portion 37, that is, the circumferential direction of the needle tube 21. At this time, the urging member 27 is compressed while being elastically deformed between the end surface of the other end of the protector 26 in the axial direction and the bottom surface 36a of the accommodation recess portion 36.

Then, the sliding protrusion 47 passes through the inclined portion 38c and moves to the other end of the second sliding portion 38b in the axial direction. Further, the depth of the groove of the second sliding portion 38b is set to be deeper than the groove depths of the first sliding portion 38a and the inclined portion 38c. For that reason, the sliding protrusion 47 moving to the second sliding portion 38b does not return to the inclined portion 38c and the first sliding portion 38a again.

Further, when the sliding groove 38 is provided with the inclined portion 38c and the protector 26 is rotated by a predetermined angle, the movement distance of the protector 26 in the axial direction of the support portion 37 can be set to be shorter than that of the case without the inclined portion 38c. Accordingly, since the length of the protector 26 in the axial direction can be shortened, the injection needle assembly 2 can be decreased in size.

Further, in a case where the above-described configuration is applied to a subcutaneous injection device in which the needle tube punctures a layer lower than the upper layer of the skin, the length of the needle tube puncturing the skin becomes longer than that of the injection needle assembly 2 which punctures the upper layer of the skin by the needle tube 21 in this example and thus the length of the protector 26 in the axial direction also increases. For that reason, the rotation distance of the protector in the circumferential direction of the support portion becomes longer than that of the injection needle assembly 2 of this example. That is, in the subcutaneous injection device, the protector needs to be largely rotated on the skin. Accordingly, there is a concern that the protector is wound with the skin so that the user feels uncomfortable.

On the contrary, since the injection needle assembly 2 of this example punctures the upper layer of the skin by the needle tip 21a of the needle tube 21, the protrusion length L of the needle tube 21 is very short to be 0.5 to 3.0 mm. For that reason, the length of the protector 26 in the axial direction can be shortened and the rotation distance of the protector 26 in the circumferential direction of the support portion 37 can be set to be very short. As a result, it is possible to reduce the discomfort given to the user even when the protector 2 6 rotates at the time of puncturing the skin by the needle tip 21a of the needle tube 21.

After the needle tip 21a of the needle tube 21 punctures the skin, the pusher member 4 (see Fig. 1) is pressed so that the gasket 13 moves toward the discharge portion. Accordingly, the drug filled in the liquid chamber 14 of the syringe 3 is extruded from the discharge portion to pass through the needle hole of the needle tube 21 and is injected from the needle tip 21a into the upper layer of the skin. At this time, since a space is not formed between the front end of the discharge portion and the proximal end of the needle tube 21, the drug remaining amount can be decreased.

When the administration of the drug is completed, the drug injection device 1 is separated from the skin and the end surface 33a of the stable portion 33, the needle protruding surface 32a, and the front end surface 26a of the protector 26 are separated from the skin. At this time, the urging member 27 is released from the pressing from the skin through the protector 26. Then, the protector 26 is urged toward the needle tip 21a of the needle tube 21 by the restoring force (the urging force) of the urging member 27.

As illustrated in Fig. 4, the sliding protrusion 47 slides insides the second sliding portion 38b of the sliding groove 38, moves beyond the return regulation portion 43, and contacts with the second stopper 42. Accordingly, the movement of the protector 26 in the axial direction is regulated. Further, it is possible to prevent the separation of the protector 26 from the support portion 37 of the first member 23.

The protector 26 covers the periphery of the needle tip 21a of the needle tube 21 and the needle tube 21 is accommodated inside the protector 26. That is, the protector 26 returns from the second position to the first position. Accordingly, since the protector 26 can be automatically moved and rotated in accordance with the puncturing operation, it is possible to easily cover the periphery of the needle tip 21a of the needle tube 21.

Further, one end surface of the return regulation portion 43 in the axial direction is uprightly formed in a substantially perpendicular direction from the bottom surface of the second slidingportion 38b. For that reason, the movement of the sliding protrusion 47 fromone side to the other side in the axial direction of the second sliding portion 38b is regulated. Thus, the movement of the protector 26 after puncturing from one side toward the other side in the axial direction is regulated. Accordingly, it is possible to prevent the needle tip 21a of the needle tube 21 after puncturing from protruding again from the front end surface 26a of the protector 26. As a result, since it is possible to safely maintain the needle tip 21a of the needle tube 21 after use, it is possible to prevent the puncturing of the needle tip 21a of the needle tube 21 after use contrary to the intension of the user.

Further, since the needle tip 21a of the needle tube 21 after use is covered by the protector 26, it is possible to prevent the scattering of the blood adhering to the needle tip 21a and thus to prevent the infection of the blood.

### 2. Second Embodiment

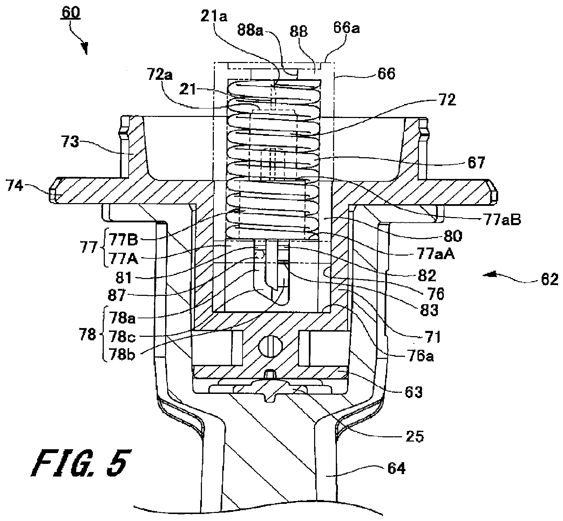

Next, a drug injection device according to a second embodiment will be described with reference to Figs. 5 to 7.

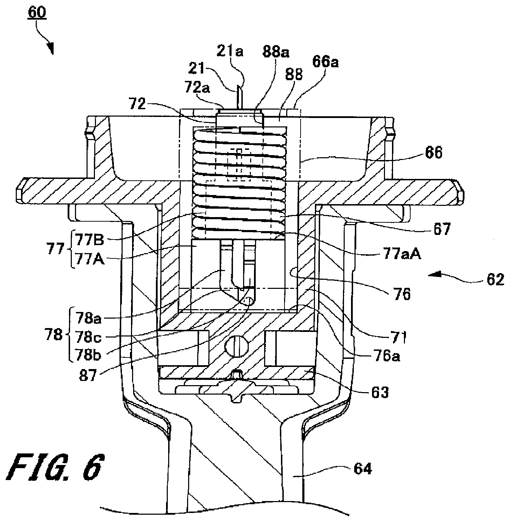

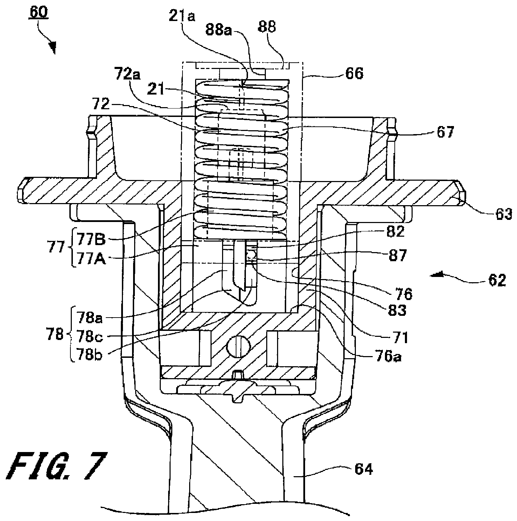

Fig. 5 is a cross-sectional view illustrating a state before puncturing is performed, Fig. 6 is a cross-sectional view illustrating a state where puncturing is performed, and Fig. 7 is a cross-sectional view illustrating a state after puncturing is performed.

The drug injection device according to the second embodiment is different from the drug injection device 1 according to the first embodiment in the configurations of the first member, the urging member, and the protector of the injection needle assembly. For that reason, the first member, the urging member, and the protector will be mainly described here. The same reference numerals will be given to the same parts as those of the injection needle assembly 2 according to the first embodiment and a repetitive description thereof will be omitted.

As illustrated in Fig. 5, an injection needle assembly 60 includes the hollow needle tube 21 and a needle hub 62 which holds the needle tube 21. The needle hub 62 includes a first member 63, a second member 64 into which the discharge portion of the syringe 3 is fitted, the elastic member 25, a protector 66, and an urging member 67.

The first member 63 is configured to include a base portion 71, an adjustment portion 72, a stable portion 73, a guide portion 74, and a support portion 77. The base portion 71 is formed in a substantially columnar shape. The base portion 71 is provided with an accommodation recess portion 76. The accommodation recess portion 76 is formed to be recessed in a substantially columnar shape from one end toward the other end in the axial direction of the base portion 71. The accommodation recess portion 76 is provided with the support portion 77.

The support portion 77 is provided at the center portion of a bottom surface 76a of the accommodation recess portion 76 and extends in the axial direction of the base portion 71 from the bottom surface 76a of the accommodation recess portion 76. The support portion 77 includes a first column portion 77A and a second column portion 77B. The first column portion 77A and the second column portion 77B are formed in a substantially columnar shape.

The first column portion 77A protrudes from the bottom surface 76a of the accommodation recess portion 76 in the axial direction of the base portion 71. The second column portion 77B is provided at the center portion of one end surface 77aA of the first column portion 77A in the axial direction and protrudes in the axial direction of the base portion 71. The axis of the first column portion 77A matches the axis of the second column portion 77B. The outer diameter of the second column portion 77B is set to be smaller than the outer diameter of the first column portion 77A.

Further, one end surface 77aB of the second column portion 77B in the axial direction is provided with the adjustment portion 72. The adjustment portion 72 is provided at the center portion of one end surface 77aB of the second column portion 77B. Then, the axis of the adjustment portion 72 matches the axes of the first column portion 77A and the second column portion 77B.

A side surface of the first column portion 77A is provided with a sliding groove 78. The sliding groove 78 includes a first sliding portion 78a, a second sliding portion 78b, and an inclined portion 78c. The first sliding portion 78a and the second sliding portion 78b extend by a predetermined length from the outer edge of one end surface 77aA of the first column portion 77A in the axial direction of the first column portion 77A.

The first sliding portion 78a is provided with a first stopper 81. The second sliding portion 78b is provided with a second stopper 82 and a return regulation portion 83. A sliding protrusion 87 of the protector 66 to be described later is inserted into the first sliding portion 78a, the second sliding portion 78b, and the inclined portion 78c to be slidable.

Next, the protector 66 will be described. The protector 66 is formed in a cylindrical shape and covers the peripheries of the second column portion 77B, the adjustment portion 72 through which the needle tube 21 passes, and the needle tip 21a of the needle tube 21 in a state before the needle tube 21 punctures the skin. The protector 66 is supported by the support portion 77 to be movable in the axial direction and rotatable in the circumferential direction. Then, a part of the protector 66 at the other side in the axial direction is inserted into a space 80 formed between the accommodation recess portion 76 and the support portion 77.

The inner wall of the cylinder hole of the protector 66 is provided with a sliding protrusion 87 and a spring contact portion 88. The spring contact portion 88 is disposed at one end of the protector 66 in the axial direction and the sliding protrusion 87 is disposed at the other end of the protector 66 in the axial direction.

The sliding protrusion 87 protrudes inward in the radial direction of the protector 66 from the inner wall of the protector 66. Then, the sliding protrusion 87 is inserted into the sliding groove 78 to be slidable.

The spring contact portion 88 is an inner flange which is formed to be continuous to the inner wall of the protector 66 in the circumferential direction and protrudes inward in the radial direction of the protector 66. An insertion hole 88a through which the adjustment portion 72 and the needle tip 21a of the needle tube 21 are inserted are provided at the inside of the spring contact portion 88 in the radial direction.

The urging member 67 is disposed to cover the peripheries of the adjustment portion 72 and the second column portion 77B of the support portion 77 inside the cylinder hole of the protector 66. One end of the urging member 67 in the axial direction contacts with the spring contact portion 88 and the other end of the urging member 67 in the axial direction contacts with one end surface 77aA of the first column portion 77A. Then, the urging member 67 urges the protector 66 toward one side in the axial direction, that is, the needle tip 21a of the needle tube 21.

Next, an operation of the protector 66 of the injection needle assembly 60 according to the second embodiment with the above-described configuration will be described.

As illustrated in Fig. 5, in a state before puncturing, the sliding protrusion 87 is disposed at one side in the axial direction of the first sliding portion 78a of the sliding groove 78 and contacts with the first stopper 81. At this time, the protector 66 covers the peripheries of the second column portion 77B, the adjustment portion 72 through which the needle tube 21 passes, and the needle tip 21a of the needle tube 21. Accordingly, it is possible to prevent the erroneous puncturing of the needle tip 21a of the needle tube 21 before the skin is punctured by the needle tip 21a of the needle tube 21.

Next, when a front end surface 66a of the protector 66 is pressed against the skin while resisting the urging force of the urging member 67, the sliding protrusion 87 slides along the first sliding portion 78a and the protector 66 moves along the support portion 77. Then, as illustrated in Fig. 6, a needle protruding surface 72a of the adjustment portion 72 and the needle tip 21a of the needle tube 21 protrude from the insertion hole 88a of the protector 66.

Further, the sliding protrusion 87 passes through the inclined portion 78c from the first slidingportion 78a and slides to the second sliding portion 78b. Then, when the sliding protrusion 87 passes through the inclined portion 78c, the protector 66 rotates about the axis of the support portion 77 in the circumferential direction of the support portion 77. At this time, the urging member 67 is compressed while being elastically deformed between the spring contact portion 88 of the protector 66 and one end surface 77aA of the first column portion 77A.

Next, the pressing of the urging member 67 from the skin through the protector 66 is released by the separation from the front end surface 66a of the protector 66. Then, the protector 66 is urged toward the needle tip 21a of the needle tube 21 by the restoring force (the urging force) of the urging member 67.

As illustrated in Fig. 7, the sliding protrusion 87 slides inside the second sliding portion 78b, goes beyond the return regulation portion 83, and contacts with the second stopper 82. Then, the protector 66 covers the periphery of the needle tip 21a of the needle tube 21 and the needle tube 21 is accommodated inside the protector 66.

Since the other configurations are the same as those of the injection needle assembly 2 according to the first embodiment, a description thereof will be omitted. Even in the injection needle assembly 60 with such a configuration, it is possible to obtain the similar operation and effect as those of the injection needle assembly 2 according to the above-described first embodiment.

Further, in the injection needle assembly 60 according to the second embodiment, the urging member 67 is accommodated in the cylinder hole of the protector 66. For that reason, the urging member 67 is disposed near the needle tip 21a of the needle tube 21 in relation to the urging member 27 of the injection needle assembly 2 according to the first embodiment. Accordingly, since the axial length of the accommodation recess portion 76 formed in the base portion 71 can be shorter than the axial length of the accommodation recess portion 36 according to the first embodiment, the first member 63 can be shorter than the first member 23 according to the first embodiment. As a result, the injection needle assembly 60 can be decreased in size.

Furthermore, the elastically deformed urging member 67 is disposed at a position separated from the sliding groove 78. For that reason, it is possible to prevent the interference between both members when the urging member 67 is elastically deformed or the sliding protrusion 87 slides in the sliding groove 78. Accordingly, the protector 66 can smoothly move and rotate.

As described above, the embodiments of the drug injection device and the injection needle assembly of the invention have been described along with the operation and effect thereof. However, the drug injection device and the injection needle assembly of the invention are not limited to the above-described embodiments and can be modified into various forms without departing from the spirit of the invention described in claims.

In the above-described embodiments, an example in which the sliding protrusion is provided in the protector and the sliding groove is provided in the support portion has been described, but the invention is not limited thereto. For example, the sliding protrusion may be provided in the support portion and the sliding groove may be provided in the protector.

Further, in the above-described embodiments, a length in which the second sliding portion extends in the axial direction is set to be longer than a length in which the first sliding portion extends in the axial direction and a depth of the groove of the second sliding portion is set to be deeper than the groove depths of the first sliding portion and the inclined portion, but the invention is not limited thereto.

For example, a length in which the second sliding portion extends in the axial direction is set to be shorter than a length in which the first sliding portion extends in the axial direction. For that reason, the inclined portion is inclined from the other side toward one side in the axial direction of the support portion as it goes from the first sliding portion to the second sliding portion in the circumferential direction of the support portion. Then, a depth of the groove of the inclined portion is set to be deeper than the groove depth of the first sliding portion and to be equal to the groove depth of the second sliding portion. Further, the groove depth of the end near the inclined portion in the first sliding portion is set to be equal to the groove depth of the inclined portion.

According to this configuration, when the protector is pressed against the skin, the sliding protrusion slides on the first sliding portion and moves along the axial direction of the support portion. Then, the sliding protrusion moves to the end near the inclinedportionin the first sliding portion. Then, when the protector is separated from the skin, the sliding protrusion slides on the inclined portion and the second sliding portion. Here, since the groove depth of the end near the inclined portion in the first sliding portion is set to be deeper than the groove depths at other positions in the first sliding portion, the sliding protrusion slides on the inclined portion while not sliding on the first sliding portion again.

Further, when the slidingprotrusion slides on the inclined portion, the protector rotates along the circumferential direction of the support portion. For that reason, since the protector rotates when the protector is separated from the skin, it is possible to reduce the discomfort of the user generated when the protector rotates on the skin.

Further, in the above-described embodiments, an example in which the lure locking portion is provided as the locking mechanism 12 has been described, but the invention is not limited thereto. For example, a configuration may be employed in which the discharge portion is provided with a male screw portion, the cylinder hole of the second member 24 of the injection needle assembly 2 is provided with a female screw portion, and both screw portions are threaded into each other.

### Reference Signs List

1 ... drug injection device, 2, 60 ... injection needle assembly, 3 ... syringe, 4 ... pusher member, 21 ... needle tube, 21a ... needle tip, 22, 62 ... needle hub, 23, 63 ... first member, 24, 64 ... second member, 26, 66 ... protector, 26a, 66a ... front end surface, 27, 67 ... urging member, 31, 71 ... base portion, 32, 72...adjustment portion, 32a, 72a...needle protruding surface, 33, 73 ... stable portion, 33a ... end surface, 34, 74 ... guide portion, 34a ... contact surface, 36, 76 ... accommodation recess portion, 36a, 76a ... bottom surface, 37, 77 ... support portion, 38, 78 ... sliding groove, 38a, 78a ... first sliding portion, 38b, 78b ... second sliding portion, 38c, 78c ... inclined portion, 40, 80 ... space, 41, 81 ... first stopper, 42, 82 ... second stopper, 43 ... return regulation portion, 47, 87 ... sliding protrusion, 77A ... first column portion, 77aA, 77aB ... one end surface, 77B ... second column portion, 88 ... spring contact portion, 88a ... insertion hole

## Claims

1. An injection needle assembly comprising:
a needle tube that includes a needle tip capable of puncturing a living body;
a needle hub that holds the needle tube;
a protector that is movable between a first position of covering the needle tip of the needle tube and a second position of exposing the needle tip of the needle tube;
an urging member that urges the protector toward the needle tip of the needle tube along the axial direction of the needle tube;
a sliding groove that is provided at one of the needle hub and the protector; and
a sliding protrusion that is provided at the other of the needle hub and the protector and is inserted into the sliding groove to be slidable,
wherein the sliding groove includes
a first sliding portion that extends along the axial direction of the needle tube,
an inclined portion that communicates with the first sliding portion and is inclined with respect to the axial direction of the needle tube and the circumferential direction of the needle tube, and
a second sliding portion that communicates with the inclined portion and extends along the axial direction of the needle tube.

2. The injection needle assembly according to claim 1,
wherein a groove depth of the first sliding portion is set to be shallower than a groove depth of the second sliding portion.

3. The injection needle assembly according to claim 1 or 2,
wherein the urging member is disposed near the needle tip in the axial direction of the needle tube in relation to the sliding groove.

4. The injection needle assembly according to any one of claims 1 to 3,
wherein the sliding protrusion sequentially slides on the first sliding portion, the inclined portion, and the second sliding portion, and
wherein the second sliding portion is provided with a return regulation portion that contacts with the sliding protrusion and regulates the movement of the protector from the first position to the second position.

5. The injection needle assembly according to any one of claims 1 to 4, comprising:
an adjustment portion that is provided in the periphery of the needle tube and includes a needle protruding surface which contacts a skin at the time of puncturing the living body by the needle tube and from which the needle tip of the needle tube protrudes,
wherein the protector covers the periphery of the adjustment portion at the first position.

6. The injection needle assembly according to any one of claims 1 to 5, comprising:
a stable portion that extends from the needle hub, is disposed to cover the peripheries of the needle tube and the protector, and includes an end surface contacting a skin at the time of puncturing a living body by the needle tube; and
a guide portion that is formed in a flange shape protruding in a substantially perpendicular direction outward in the radial direction from an outer peripheral surface of the stable portion.

7. A drug injection device comprising:
an injection needle assembly that includes a needle tube with a needle tip capable of puncturing a living body; and
a syringe that is separably attached to the injection needle assembly,
wherein the injection needle assembly includes
a needle hub that holds the needle tube,
a protector that is movable between a first position of covering the needle tip of the needle tube and a secondposition of exposing the needle tip of the needle tube,
an urging member that urges the protector toward the needle tip of the needle tube along the axial direction of the needle tube,
a sliding groove that is provided at one of the needle hub and the protector, and
a sliding protrusion that is provided at the other of the needle hub and the protector and is inserted into the sliding groove to be slidable,
wherein the sliding groove includes
a first sliding portion that extends along the axial direction of the needle tube,
an inclined portion that communicates with the first sliding portion and is inclined with respect to the axial direction of the needle tube and the circumferential direction of the needle tube, and
a second sliding portion that communicates with the inclined portion and extends along the axial direction of the needle tube.
